(19) **Europäisches Patentamt**
**European Patent Office**
**Office européen des brevets**

(11) **EP 4 591 897 A1**

(12) **EUROPÄISCHE PATENTANMELDUNG**

(43) Veröffentlichungstag:
30.07.2025  Patentblatt 2025/31

(21) Anmeldenummer: 25153435.0

(22) Anmeldetag: 22.01.2025

(51) Internationale Patentklassifikation (IPC):
**A61M 1/16** (2006.01)

(52) Gemeinsame Patentklassifikation (CPC):
**A61M 1/1603; A61M 1/1658;** A61M 2205/3331;
A61M 2230/205; A61M 2230/207

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(30) Priorität: **29.01.2024  DE 102024102454**

(71) Anmelder: **B. Braun Avitum AG
34212 Melsungen (DE)**

(72) Erfinder: **WAGNER, Andre
34117 Kassel (DE)**

(74) Vertreter: **Winter, Brandl - Partnerschaft mbB
Alois-Steinecker-Straße 22
85354 Freising (DE)**

(54) **EXTRAKORPORALE BLUTBEHANDLUNGSMASCHINE, COMPUTERIMPLEMENTIERTES STEUERVERFAHREN FÜR DIESE, SOWIE COMPUTERPROGRAMM**

(57)    Die Offenbarung betrifft eine extrakorporale Blutbehandlungsmaschine (1) für eine extrakorporale Blutbehandlung, aufweisend einen Dialysator (2), einen extrakorporalen Blutkreislauf (3), eine Sensoreinheit (44), die dafür angepasst ist, einen Blutbestandteil $(SpO_{2B})$ in dem Blut zu ermitteln, eine Entgasungseinheit (5) mit einer Drosseleinrichtung (78), die dafür angepasst ist, gashaltiges Reinstwasser auf einen Entgasungs-Druck $(p_E)$ zu drosseln, eine Druckerfassungseinheit (81), die dafür angepasst ist, den Entgasungs-Druck $(p_E)$ zu erfassen, eine Mischeinheit (6), die dafür angepasst ist, das zumindest teilentgaste Reinstwasser mit wenigstens einem Konzentrat zu einer frischen Dialysierflüssigkeit zu mischen, und eine Steuereinheit (54), die mit der Sensoreinheit (44), der Druckerfassungseinheit (81) und der Drosseleinrichtung (78) signalverbunden ist, wobei die Drosseleinrichtung (78) mit kontinuierlich verstellbarem Drosselquerschnitt ausgestaltet ist, der in Abhängigkeit eines Ansteuersignals (S) der Steuereinheit (54) kontinuierlich verstellbar ist. Daneben betrifft die Offenbarung ein Steuerverfahren und ein Computerprogramm gemäß den nebengeordneten Ansprüchen.

Fig. 1

**Beschreibung**

**Technisches Gebiet**

Die vorliegende Offenbarung betrifft eine extrakorporale Blutbehandlungsmaschine, insbesondere eine Dialysemaschine, für eine extrakorporale Blutbehandlung wie etwa eine Hämodialyse, eine Hämofiltration, eine Hämodiafiltration und/oder eine Ultrafiltration. Die Blutbehandlungsmaschine hat einen Dialysator mit einer semipermeablen Membran für einen Stoffaustausch zwischen einem Blut eines Patienten und einer Dialysierflüssigkeit, einen extrakorporalen Blutkreislauf, welcher über einen Bluteingang und einen Blutausgang des Dialysators durch den Dialysator verläuft, eine Sensoreinheit, um einen Blutbestandteil in dem Blut zu ermitteln, der mit einem Sauerstoffgehalt in dem Blut korreliert, eine Entgasungseinheit mit einer Drosseleinrichtung, über die gashaltig bereitgestelltes Reinstwasser auf einen Entgasungs-Druck gedrosselt werden kann, um das zumindest teilentgaste Reinstwasser an einem Ausgang der Entgasungseinheit bereitzustellen, eine Druckerfassungseinheit, über die der Entgasungs-Druck erfasst wird, eine Mischeinheit, in der das zumindest teilentgaste Reinstwasser mit wenigstens einem Konzentrat zu einer frischen Dialysierflüssigkeit gemischt wird und einem Dialysierflüssigkeitskreislauf der Blutbehandlungsmaschine bereitgestellt wird, und eine Steuereinheit, die mit der Sensoreinheit, der Druckerfassungseinheit und der Drosseleinrichtung signalverbunden ist. Daneben betrifft die vorliegende Offenbarung ein computerimplementiertes Steuerverfahren für eine extrakorporale Blutbehandlungsmaschine sowie ein Computerprogramm gemäß den Oberbegriffen der nebengeordneten Ansprüche.

**Technischer Hintergrund**

Bei einer extrakorporalen Blutbehandlung, beispielsweise einer Blutreinigung in Form einer Hämodialyse, Hämofiltration oder Hämodiafiltration, wird einem Dialysepatienten Blut über einen arteriellen Gefäßzugang entnommen und über einen extrakorporalen Blutkreislauf einem Dialysator für eine Blutbehandlung zugeführt. Dem Dialysator wird zudem bedarfsgerecht hergestellte, frische Dialysierflüssigkeit über einen Dialysierflüssigkeitskreislauf zugeführt. Zur Herstellung der frischen Dialysierflüssigkeit wird von einer Wasseraufbereitung, insbesondere von einer Umkehrosmoseanlage, Reinstwasser bereitgestellt, entgast und in Folge in einer Mischeinheit mit wenigstens einem Konzentrat gemischt. Herkömmliche Blutbehandlungs-/ Dialysemaschinen entgasen derzeit vollständig, insbesondere um eine exakte Ultrafiltration sicherstellen zu können. Die Entgasung erfolgt, indem das Reinstwasser durch eine feste Drossel strömt, dadurch einen Druckverlust erfährt, bzw. entspannt wird, und im Anschluss durch eine Entgasungskammer mit großer Oberfläche geleitet wird. An der Oberfläche kann die Abscheidung der aufgrund des Druckverlustes entlösten Gasbestandteile des Reinstwassers effizient erfolgen. Das so entgaste Reinstwasser tritt in Folge in die o.g. Mischeinheit zur Herstellung der Dialysierflüssigkeit ein.

In dem Dialysator werden das Blut des extrakorporalen Blutkreislaufs und die Dialysierflüssigkeit des Dialysierflüssigkeitskreislaufs über die semipermeable Membran in Kontakt gebracht, so dass ein Stoffaustausch zwischen dem Blut und der Dialysierflüssigkeit stattfinden kann. So können bei der Dialysebehandlung niereninsuffizienter Patienten Schadstoffe aus dem Blut sowie auch überschüssiges Wasser, welches sich aufgrund eines zugrundeliegenden Nierenversagens im Körper ansammelt, entfernt werden. Das so gereinigte Blut wird anschließend über einen venösen Gefäßzugang an den Patienten wieder zurückgeführt.

Hierbei ist zu beachten, dass der Stoffaustausch zwischen dem Blut des extrakorporalen Blutkreislaufs und der Dialysierflüssigkeit in beide Richtungen stattfinden kann. Eine resultierende Richtung des Stofftransports hängt dabei vom Gehalt des betreffenden Bestandteils in dem Blut/ in der Dialysierflüssigkeit ab. Für die Hämodialyse ist der Sauerstoffgehalt der Dialysierflüssigkeit bedeutsam, da Sauerstoff gut permeabel ist und bei ausreichender Partialdruckdifferenz zwischen dem Blut des extrakorporalen Blutkreislaufs und der frischen Dialysierflüssigkeit, je nach Gradient, mit dem Stofftransport gerechnet werden muss.

Da aufgrund der vollständigen Entgasung der nach dem aktuellen Stand der Technik ausgeführten Blutbehandlungsmaschine der Partialdruck des Sauerstoffs in der frischen Dialysierflüssigkeit gegen null geht und die frische Dialysierflüssigkeit an der semipermeablen Membran des Dialysators mit dem sauerstoffhaltigen Blut des extrakorporalen Blutkreislaufs in Kontakt gebracht wird, ist nach dem aktuellen Stand der Technik mit der vermehrten Ausscheidung von im Blut des extrakorporalen Blutkreislaufs gelöstem Sauerstoff und in Folge mit einer Hypoxämie des venösen Blutes im extrakorporalen Blutkreislauf zu rechnen. Dieser nachteilige Effekt kann vermieden oder zumindest gemindert werden, sollte es gelingen, den Partialdruck in dem Blut des extrakorporalen Blutkreislaufs zu beeinflussen.

Verfahren zur Beeinflussung eines Gasbestandteils in einem extrakorporalen Blutkreislauf sind grundlegend aus dem Stand der Technik bekannt. So kombiniert beispielsweise eine Membranoxygenierung eine kontinuierliche Nierenersatztherapie (CRRT) mit einer venovenösen, extrakorporalen Entfernung von Kohlendioxid $CO_2$ (ECCO2R). Hierbei wird mittels einer Peristaltikpumpe Blut eines arteriellen Schlauchabschnitts des extrakorporalen Blutkreislaufs mit einem Fluss von etwa 10 ml/min bis 500 ml/min in einen $CO_2$ Absorber (ECCO2R-Filter) gefördert. Der $CO_2$-Absorber

hat einen Sauerstoff-Druckanschluss, der von einem externen Sauerstoffbehälter gespeist wird. Der so bereitgestellte Sauerstoff-Spülgasstrom entfernt aus dem Blut des arteriellen Schlauchabschnitts das $CO_2$.

Nachteilig an dieser Lösung ist, dass der notwendige $CO_2$-Absorber, der Sauerstoffbehälter und das Leitungssystem für die Zu- und Abfuhr des Spülsauerstoffs einen vergleichsweise hohen vorrichtungstechnischen Aufwand darstellen, was sich in Kosten und in einem hohen Platzbedarf wiederspiegelt.

**Zusammenfassung der vorliegenden Offenbarung**

Die Aufgabe der vorliegenden Offenbarung ist demgegenüber, die Nachteile aus dem Stand der Technik zu vermeiden oder zumindest zu mindern und insbesondere eine extrakorporale Blutbehandlungsmaschine, sowie ein computerimplementiertes Steuerverfahren und Computerprogramm zur Verfügung zu stellen, welche(s) eine noch effizientere und sicherere Therapie einer extrakorporalen Blutbehandlung bereitstellt.

Die Aufgabe der vorliegenden Offenbarung wird hinsichtlich einer extrakorporalen Blutbehandlungsmaschine offenbarungsgemäß durch die Merkmale des Anspruchs 1 gelöst, hinsichtlich eines computerimplementierten Steuerverfahrens offenbarungsgemäß durch die Merkmale des Anspruchs 10 gelöst und hinsichtlich eines Computerprogramms offenbarungsgemäß durch die Merkmale des Anspruchs 15 gelöst.

Ein Grundgedanke der vorliegenden Offenbarung sieht vor, dass eine Blutbehandlungsmaschine dafür angepasst ist, einen Sauerstoffgehalt in dem Blut während der Blutbehandlung durch einen Sauerstoffgehalt in der frischen Dialysierflüssigkeit zu beeinflussen. Hierzu wird ein Entgasungsdruck von gashaltigem Reinstwasser, das zur Herstellung der frischen Dialysierflüssigkeit bereitgestellt wird, mittels einer kontinuierlich verstellbaren Drosseleinrichtung gezielt verstellt oder eingestellt.

Mit anderen Worten wird eine extrakorporale Blutbehandlungsmaschine, insbesondere Dialysemaschine, für eine extrakorporale Blutbehandlung von Blut eines Patienten bereitgestellt, die aufweist:

einen Dialysator;

einen extrakorporalen Blutkreislauf, welcher über einen Bluteingang und einen Blutausgang des Dialysators durch den Dialysator verläuft, mit einer Sensoreinheit, die dafür angepasst ist, einen Blutbestandteil in dem Blut zu ermitteln, insbesondere einen Oxyhämoglobinanteil des Blutes, welcher mit einem Sauerstoffgehalt in dem Blut, insbesondere einem Sauerstoff-Partialdruck in dem Blut, korreliert, wobei die Sensoreinheit vorzugsweise stromaufwärts des Bluteingangs angeordnet ist;

eine Entgasungseinheit mit einer Drosseleinrichtung, die dafür angepasst ist, gashaltiges Reinstwasser auf einen Entgasungs-Druck zu drosseln, um an einem Ausgang der Entgasungseinheit zumindest teilentgastes Reinstwasser bereitzustellen, mit einer Druckerfassungseinheit, die dafür angepasst ist, den Entgasungs-Druck zu erfassen, wobei die Druckerfassungseinheit insbesondere stromabwärts der Drosseleinrichtung angeordnet ist,

eine Mischeinheit, die dafür angepasst ist, das zumindest teilentgaste Reinstwasser mit wenigstens einem Konzentrat zu einer frischen Dialysierflüssigkeit zu mischen,

vorzugsweise einen Dialysierflüssigkeitskreislauf, der angepasst ist, die frische Dialysierflüssigkeit an einem Dialysierflüssigkeitseingang bereitzustellen, durch den Dialysator zu fördern und an einem Dialysierflüssigkeitsausgang verbrauchte Dialysierflüssigkeit abzuführen, und

eine Steuereinheit, die mit der Sensoreinheit, der Druckerfassungseinheit und der Drosseleinrichtung signalverbunden ist.

Offenbarungsgemäß ist die Drosseleinrichtung mit einem kontinuierlich verstellbaren Drosselquerschnitt ausgestaltet, welcher in Abhängigkeit eines Ansteuersignals der Steuereinheit, vorzugsweise proportional zu dem Ansteuersignal, kontinuierlich verstellbar ist.

Anders als aus dem Stand der Technik bekannt, ist der Drosselquerschnitt der Drosseleinrichtung somit offenbarungsgemäß kontinuierlich verstellbar. In anderen Worten ausgedrückt, kann der Drosselquerschnitt in eine vollständige Offenstellung und eine vollständige Sperrstellung und in dem Ansteuersignal entsprechende Zwischenstellungen verstellt/ eingestellt werden. Da der Entgasungs-Druck von einem Druckverlust über die Drosseleinrichtung abhängt, und der Druckverlust wiederum - bei gegebenem Volumenstrom des in die Drosseleinrichtung eintretenden Reinstwassers - im Wesentlichen von dem Drosselquerschnitt bestimmt ist, kann der Entgasungs-Druck offenbarungsgemäß zwischen Grenzen kontinuierlich verstellt und beeinflusst werden. Da die Löslichkeit von Gasen in einer Flüssigkeit - und damit auch von Sauerstoff - gemäß dem Henry-Gesetz direkt von dem Druck der Flüssigkeit abhängig ist (im vorliegenden Fall ist dieser Druck der Entgasungs-Druck des Reinstwassers stromabwärts der Drosseleinrichtung) ist in der Konsequenz durch den offenbarungsgemäß kontinuierlich verstellbaren Drosselquerschnitt ein Sauerstoffgehalt des Reinstwassers stromabwärts der Drosseleinrichtung verstellbar/ einstellbar. Da dieses zumindest teilentgaste Reinstwasser in der Mischeinheit mit einem Konzentrat zu frischer Dialysierflüssigkeit gemischt wird, kann so offenbarungsgemäß der Sauerstoffgehalt der frischen Dialysierflüssigkeit verstellt/ eingestellt werden. Da die frische Dialysierflüssigkeit im Dialysator über die semipermeable Membran des Dialysators in Stoffaustausch mit dem Blut steht und Sauerstoff membrangängig ist, eröffnet sich hierin die Möglichkeit, über den verstellten/ eingestellten Sauerstoffgehalt der frischen Dialysierflüssigkeit den Sauerstoffgehalt in dem Blut zu beeinflussen. Dadurch ist mittels der offenbarungsgemäß ausgestalteten Blutbehandlungsmaschine eine noch effizientere und sicherere Therapie einer extrakorporalen Blutbehandlung bereitgestellt.

Eine untere Grenze des Entgasungs-Drucks ist mit minimal aufgesteuertem Drosselquerschnitt erreichbar, wodurch eine maximale, insbesondere vollständige, Entgasung des gashaltigen Reinstwassers möglich ist. Eine obere Grenze des Entgasungs-Drucks ist demgegenüber mit maximal aufgesteuertem Drosselquerschnitt erreichbar, sodass keine Entgasung des gashaltigen Reinstwassers oder lediglich eine minimale Entgasung, insbesondere aufgrund eines ohnehin auftretenden Strömungsdruckverlustes, erfolgt.

Bevorzugt ist die Drosseleinrichtung als ein elektromagnetisch betätigbares Proportionalventil ausgestaltet oder hat ein solches und ist mit der Steuereinheit signalverbunden.

In bevorzugter Weiterbildung ist die Steuereinheit dafür angepasst, das Ansteuersignal so zu ermitteln, dass in Folge der Verstellung des Drosselquerschnitts der Sauerstoffgehalt in dem Blut wahlweise angehoben, konstant gehalten und/oder abgesenkt wird, sodass auf den Sauerstoffgehalt in dem Blut gezielt Einfluss genommen wird.

In weiterer, bevorzugter Weiterbildung ist die Steuereinheit dafür angepasst, das Ansteuersignal so zu ermitteln, dass ein Sauerstoffgehalt in dem zumindest teilentgasten Reinstwasser oder der frischen Dialysierflüssigkeit, insbesondere ein Sauerstoff-Partialdruck in dem zumindest teilentgasten Reinstwasser oder der frischen Dialysierflüssigkeit, in Abhängigkeit des Sauerstoffgehaltes in dem Blut eingestellt wird, insbesondere angehoben, konstant gehalten und/oder abgesenkt wird.

In weiterer, bevorzugter Weiterbildung ist die Steuereinheit dafür angepasst, das Ansteuersignal zu ermitteln, dass eine vorbestimmte Differenz zwischen einem Sauerstoffgehalt in dem zumindest teilentgasten Reinstwasser oder der frischen Dialysierflüssigkeit, insbesondere einem Sauerstoff-Partialdruck in dem zumindest teilentgasten Reinstwasser oder der frischen Dialysierflüssigkeit, und dem Sauerstoffgehalt in dem Blut eingestellt, insbesondere konstant gehalten wird.

Zur Ermittlung des Ansteuersignals ist gemäß einer bevorzugten Weiterbildung in einem Speicher der Blutbehandlungsmaschine, vorzugsweise in einem Speicher der Steuereinheit, eine erste Korrelation des Sauerstoffgehalts in dem Blut mit dem Blutbestandteil abgelegt. Zudem ist die Steuereinheit dafür angepasst, die erste Korrelation aufzurufen und hieraus den Sauerstoffgehalt in dem Blut in Abhängigkeit des von der Sensoreinheit ermittelten Blutbestandteils zu ermitteln.

Zur Ermittlung des Ansteuersignals ist des Weiteren gemäß einer bevorzugten Weiterbildung die Steuereinheit dafür angepasst, aus dem ermittelten Sauerstoffgehalt in dem Blut und der vorbestimmten Differenz, insbesondere als Summe aus Sauerstoffgehalt in dem Blut und vorbestimmter Differenz, einen Soll-Sauerstoffgehalt in dem zumindest teilentgasten Reinstwasser oder der frischen Dialysierflüssigkeit zu ermitteln.

Gemäß einer bevorzugten Weiterbildung ist in dem Speicher eine zweite Korrelation des Sauerstoffgehaltes in dem zumindest teilentgasten Reinstwasser oder der frischen Dialysierflüssigkeit mit dem Entgasungsdruck abgelegt, und die Steuereinheit ist dafür angepasst, die zweite Korrelation aufzurufen und hieraus einen Soll-Entgasungsdruck in Abhängigkeit des ermittelten Soll-Sauerstoffgehaltes in dem zumindest teilentgasten Reinstwasser oder der frischen Dialysierflüssigkeit zu ermitteln.

Gemäß einer bevorzugten Weiterbildung ist in dem Speicher eine dritte Korrelation des Entgasungsdrucks mit dem Ansteuersignal abgelegt, und die Steuereinheit ist dafür angepasst, die dritte Korrelation aufzurufen und hieraus das Ansteuersignal in Abhängigkeit des ermittelten Soll-Entgasungsdrucks zu ermitteln und die Drosseleinrichtung mit dem Ansteuersignal anzusteuern.

Gemäß einer weiteren bevorzugten Weiterbildung ist die Steuereinheit dafür angepasst, eine Abweichung von der vorbestimmten Differenz permanent, punktuell, und/oder periodisch zu ermitteln und das Ansteuersignal in Abhängigkeit der Abweichung anzupassen.

Gemäß einer möglichen Weiterbildung ist die extrakorporale Blutbehandlungsmaschine dafür angepasst, eine automatische Angleichung der Sauerstoff-Partialdrücke in dem Blut und in der frischen Dialysierflüssigkeit in der chronischen Dialysebehandlung durchzuführen.

Gemäß einer weiteren möglichen Weiterbildung ist die extrakorporale Blutbehandlungsmaschine dafür angepasst, eine Einstellung des Oxyhämoglobinwertes in dem Blut in der chronischen Dialysebehandlung durchzuführen.

Gemäß einer weiteren möglichen Weiterbildung ist die extrakorporale Blutbehandlungsmaschine dafür angepasst, eine Unterstützung einer Sauerstoffversorgung in der der akuten und der chronischen Dialysebehandlung durchzuführen.

Hinsichtlich eines computerimplementierten Steuerverfahrens für eine extrakorporale Blutbehandlungsmaschine, insbesondere einer Blutbehandlungsmaschine gemäß der vorliegenden Offenbarung, wird die Aufgabe dadurch gelöst, dass das Steuerverfahren Schritte aufweist:

Ermitteln eines Blutbestandteils in dem Blut, insbesondere eines Oxyhämoglobinanteils in dem Blut, welcher mit einem Sauerstoffgehalt in dem Blut, insbesondere einem Sauerstoff-Partialdruck in dem Blut, korreliert, über eine Sensoreinheit in einem extrakorporalen Blutkreislauf der Blutbehandlungsmaschine und Bereitstellen des ermittelten Blutbestandteils an eine Steuereinheit, insbesondere eine Steuereinheit der extrakorporalen Blutbehandlungsmaschine;

Drosseln von gashaltigem Reinstwasser auf einen Entgasungs-Druck, um zumindest teilentgastes Reinstwasser bereitzustellen, über eine Drosseleinrichtung, insbesondere eine Drosseleinrichtung einer Entgasungseinheit der extrakorporalen Blutbehandlungsmaschine;

Erfassen des Entgasungs-Drucks über eine Druckerfassungseinheit, insbesondere der Entgasungseinheit, und Bereitstellen des Entgasungs-Drucks an die Steuereinheit;

Mischen des zumindest teilentgasten Reinstwassers mit wenigstens einem Konzentrat zu einer frischen Dialysierflüssigkeit, über eine Mischeinheit, insbesondere eine Mischeinheit der extrakorporalen Blutbehandlungsmaschine, Offenbarungsgemäß weist das Verfahren Schritte auf:

Ermitteln eines Ansteuersignals zum Ansteuern der Drosselvorrichtung, die mit einem in Abhängigkeit des Ansteuersignals verstellbaren, vorzugsweise proportional zu dem Ansteuersignal verstellbaren, Drosselquerschnitt ausgestaltet ist, über die Steuereinheit;

Ansteuern der Drosselvorrichtung mit dem Ansteuersignal, über die Steuereinheit; und

Verstellen des Drosselquerschnitts gemäß dem Ansteuersignal.

Gemäß einer bevorzugten Weiterbildung wird das Ansteuersignal über die Steuereinheit derart ermittelt, dass in Folge der Verstellung des Drosselquerschnitts der Sauerstoffgehalt in dem Blut wahlweise gehoben, konstant gehalten und/oder gesenkt wird.

Die Vorteile, die sich dabei aus der offenbarungsgemäßen Ermittlung des Ansteuersignals, der Ansteuerung der Drosseleinrichtung mit dem Ansteuersignal und der gemäß dem Ansteuersignal kontinuierlichen Verstellung des Drosselquerschnitts der Drosseleinrichtung ergeben, wurden bereits weiter oben für die Blutbehandlungsmaschnine offenbart, worauf an dieser Stelle verwiesen wird, sodass auf deren erneute Nennung verzichtet werden kann, um diese Schrift nicht zu überfrachten. Mit dem offenbarungsgemäßen, computerimplementierten Steuerungsverfahren ist somit eine noch effizientere und sicherere Therapie einer extrakorporalen Blutbehandlung bereitgestellt.

Gemäß einer bevorzugten Weiterbildung wird das Ansteuersignal über die Steuereinheit derart ermittelt, dass ein Sauerstoffgehalt in dem zumindest teilentgasten Reinstwasser oder der frischen Dialysierflüssigkeit, insbesondere ein Sauerstoff-Partialdruck in dem zumindest teilentgasten Reinstwasser oder der frischen Dialysierflüssigkeit, in Abhängigkeit des Sauerstoffgehaltes in dem Blut wahlweise gehoben, konstant gehalten und/oder gesenkt wird.

Gemäß einer bevorzugten Weiterbildung wird das Ansteuersignal über die Steuereinheit derart ermittelt, dass eine vorbestimmte Differenz zwischen einem Sauerstoffgehalt in dem zumindest teilentgasten Reinstwasser oder der frischen Dialysierflüssigkeit, insbesondere einem Sauerstoff-Partialdruck in dem zumindest teilentgasten Reinstwasser oder der frischen Dialysierflüssigkeit, und dem Sauerstoffgehalt in dem Blut eingestellt wird, insbesondere konstant gehalten wird.

Gemäß einer besonders bevorzugten Weiterbildung des computerimplementierten Steuerverfahrens sind in einem Speicher der Blutbehandlungsmaschine, vorzugsweise in einem Speicher der Steuereinheit, eine erste Korrelation des Sauerstoffgehalts in dem Blut mit dem Blutbestandteil, eine zweite Korrelation des Sauerstoffgehalts in dem zumindest teilentgasten Reinstwasser oder der frischen Dialysierflüssigkeit mit dem Entgasungsdruck, eine dritte Korrelation des Entgasungsdrucks mit dem Ansteuersignal, sowie die vorbestimmte Differenz abgelegt. Offenbarungsgemäß kann dann der Schritt Ermitteln des Ansteuersignals mit folgenden Schritten erfolgen:

Aufrufen der ersten Korrelation und Ermitteln des Sauerstoffgehalts in dem Blut in Abhängigkeit des von der Sensoreinheit erfassten Blutbestandteils, über die Steuereinheit;

Ermitteln eines Soll-Sauerstoffgehalts in dem zumindest teilentgasten Reinstwasser oder der frischen Dialysierflüssigkeit aus dem ermittelten Sauerstoffgehalt in dem Blut und der vorbestimmten Differenz, insbesondere als deren Summe, über die Steuereinheit;

Aufrufen der zweiten Korrelation und Ermitteln eines Soll-Entgasungsdrucks in Abhängigkeit des ermittelten Soll- Sauerstoffgehalts in dem zumindest teilentgasten Reinstwasser oder der frischen Dialysierflüssigkeit, über die Steuereinheit; und

Aufrufen der dritten Korrelation und Ermitteln des Ansteuersignals in Abhängigkeit des ermittelten Soll-Entgasungsdrucks, über die Steuereinheit.

Hinsichtlich eines Computerprogramms wird die Aufgabe der vorliegenden Offenbarung dadurch gelöst, dass dieses Computerprogramm Befehle umfasst, die bei einer Ausführung durch einen Computer diesen veranlassen, die Verfahrensschritte des Steuerverfahrens gemäß der vorliegenden Offenbarung auszuführen.

**Kurzbeschreibung der Figuren**

Die Offenbarung wird nachfolgend anhand bevorzugter Ausführungsformen mit Hilfe von Figuren näher erläutert. Es zeigen:

Fig. 1 eine schematische Ansicht einer extrakorporalen Blutbehandlungsmaschine gemäß einer bevorzugten Ausführungsform;

Fig. 2 eine erste Korrelation eines Sauerstoffgehalts $pO_{2B}$ in dem Blut eines extrakorporalen Blutkreislaufs mit einem von einer Sensoreinheit in dem Blut des extrakorporalen Blutkreislaufs erfassten Blutbestandteil $SpO_{2B}$, gemäß der extrakorporalen Blutbehandlungsmaschine gemäß Figur 1;

Fig. 3 einen zeitlichen Verlauf des Sauerstoffgehalts $pO_{2B}$ in dem Blut des extrakorporalen Blutkreislaufs im Falle eines konstanten Sauerstoffgehalts $pO_{2B}$ in einer frischen Dialysierflüssigkeit, gemäß der extrakorporalen Blutbehandlungsmaschine gemäß Figur 1 ;

Fig. 4 eine zweite Korrelation eines Entgasungs-Drucks $p_E$ von Reinstwasser mit dem Sauerstoffgehalt $pO_{2D}$ in dem Reinstwasser oder der frischen Dialysierflüssigkeit, gemäß der extrakorporalen Blutbehandlungsmaschine gemäß Figur 1;

Fig.5 eine dritte Korrelation eines Ansteuersignals S für eine Drosseleinrichtung mit dem Entgasungs-Druck $p_E$ des Reinstwassers, gemäß der extrakorporalen Blutbehandlungsmaschine gemäß Figur 1, und

Fig. 6 ein Flussdiagramm eines computerimplementierten Steuerverfahrens gemäß einer bevorzugten Ausführungsform.

Die Figuren sind schematischer Natur und sollen nur dem Verständnis der Offenbarung dienen. Gleiche Elemente sind mit denselben Bezugzeichen versehen. Merkmale verschiedener Ausführungsformen können untereinander ausgetauscht werden.

**Detaillierte Beschreibung bevorzugter Ausführungsformen**

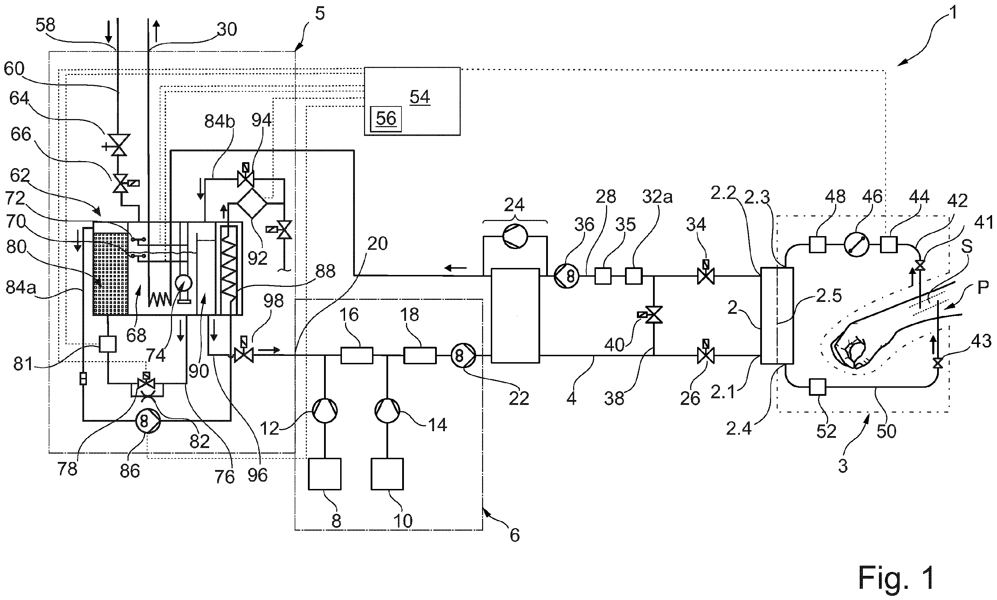

Figur 1 zeigt in einer schematischen Ansicht eine extrakorporale Blutbehandlungsmaschine 1 in Form einer Dialysemaschine für eine extrakorporale Blutbehandlung von Blut eines Patienten P gemäß einer bevorzugten Ausführungsform der vorliegenden Offenbarung.

Im Folgenden wird eine offenbarungsgemäße Verstellung oder Einstellung eines Entgasungs-Drucks $p_E$ von Reinstwasser erläutert, wodurch ein Sauerstoffgehalt $pO_{2D}$ in einer frischen Dialysierflüssigkeit der Blutbehandlungsmaschine 1 variierbar ist und in Folge, auf Basis eines Stoffaustauschs an einer semipermeablen Membran eines Dialysators 2 der Blutbehandlungsmaschine 1, ein Sauerstoffgehalt $pO_{2D}$ in dem Blut eines extrakorporalen Blutkreislaufs 3 der Blutbehandlungsmaschine 1 beeinflussbar ist.

Die extrakorporale Blutbehandlungsmaschine 1 (nachfolgend nur Blutbehandlungsmaschine genannt) weist als zentrale Komponente einen Dialysator 2 mit einerseits je einem dialysierflüssigkeitsseitigen Dialysierflüssigkeitseingang 2.1 und Dialysatausgang 2.2 und andererseits je einem blutseitigen Bluteingang 2.3 und Blutausgang 2.4 eines extrakorporalen Blutkreislaufs 3 auf. Innerhalb ist der Dialysator 2 mittels Hohlfasern einer semipermeablen Membran 2.5 in eine Dialysierflüssigkeitsseite und eine Blutseite aufgeteilt.

Der Dialysierflüssigkeitseingang 2.1 ist über einen Dialysierflüssigkeitszulauf 4 mit einer Mischeinheit 6 fluidisch verbindbar, insbesondere verbunden. Diese stellt kontinuierlich aus zumindest teilentgastem Reinstwasser, sowie einem basischen Konzentrat und einem sauren Konzentrat, frische Dialysierflüssigkeit her. Zugabemengen werden hierbei von Messvorrichtungen kontrolliert. Dementsprechend hat die Mischeinheit 6 eine erste und zweite Quelle 8, 10 für basisches und für saures Konzentrat, eine erste und zweite Fördervorrichtung 12, 14 und stromabwärts der Fördervorrichtungen 12, 14 jeweils eine erste und zweite Messvorrichtung 16, 18. Gemäß Figur 1 hat die Mischeinheit 6 einen Eingang 20, an dem das zumindest teilentgaste Reinstwasser einer - weiter unten detailliert erläuterten Entgasungseinheit 5 - ansteht. Die Mischeinheit 6 hat stromabwärts der zweiten Messvorrichtung 18 eine dritte Fördereinrichtung 22, über die die gemischte, frische Dialysierflüssigkeit zu einer Bilanzierungsvorrichtung 24 gefördert wird. Ausgangsseitig ist die Bilanzierungsvorrichtung 24 über den Dialysierflüssigkeitszulauf 4 fluidisch mit dem Dialysierflüssigkeitseingang 2.1 des Dialysators 2 verbindbar, wobei im Dialysierflüssigkeitszulauf 4 ein Ventil 26 zum Absperren des Dialysierflüssigkeitseingangs 2.1 angeordnet ist.

Der Dialysatausgang 2.2 ist über einen Dialysatablauf 28 mit einem Entsorgungsausgang 30 für verbrauchte Dialysierflüssigkeit/ Dialysat fluidisch verbindbar, insbesondere verbunden. Im Dialysatablauf 28 sind, zwischen dem Dialysatausgang 2.2 und dem Entsorgungsausgang 30, fluidisch in Reihe angeordnet: ein betätigbares Ventil 34 zum Absperren des Dialysatausgangs 2.2, eine Erfassungseinheit 32a zur Erfassung eines Bestandteils im Dialsat und eine vierte Fördervorrichtung 36, über die das Dialysat zur Bilanzierungsvorrichtung 24 und zum Entsorgungsausgang 30 für Dialysat gefördert wird. Die Bilanzierungsvorrichtung 24 sorgt dafür, dass ein gewünschtes Volumen an überschüssigem Wasser im Rahmen einer Ultrafiltration aus dem Patientenblut entzogen werden kann. Stromaufwärts der vierten Fördervorrichtung 36 ist im Dialysatablauf 28 eine Druckerfassungseinheit 35 zur Erfassung eines Dialysatausgangs-Drucks vorgesehen.

Ergänzend ist ein Bypass-Strömungspfad 38 vorgesehen, über den der Dialysierflüssigkeitszulauf 4 mit dem Dialysatablauf 28 fluidisch verbindbar ist. In dem Bypass-Strömungspfad 38 ist ein betätigbares Ventil 40 angeordnet, über das der Bypass-Strömungspfad 38 sperrbar ist.

Mit Hilfe der genannten, fluidischen Schaltmittel/ Ventile 26, 34 und 40 kann der Dialysierflüssigkeitskreislauf über die Steuereinheit 54 in eine Hauptschluss-Schaltung geschaltet werden, in der über den Dialysierflüssigkeitszulauf 4 am Dialysierflüssigkeitseingang 2.1 frische Dialysierflüssigkeit bereitgestellt wird und durch den Dialysator 2 zum Dialysatausgang 2.2 gefördert wird. Des Weiteren ist der Dialysierflüssigkeitskreislauf mittels der fluidischen Schaltmittel/ Ventile 26, 34 und 40 in eine Bypass-Schaltung schaltbar, in der jeweils der Dialysierflüssigkeitszulauf 4 vom Dialysierflüssigkeitseingang 2.1 und der Dialysatablauf 24 vom Dialysatausgang 2.2 fluidisch getrennt ist, während der Dialysierflüssigkeitszulauf 4 über den Bypass-Strömungspfad 38 mit dem Dialysatablauf 24 fluidisch verbunden ist.

Auf der Seite des Blutes ist der extrakorporale Blutkreislauf 3 vorgesehen, welcher über einen arteriellen Schlauchabschnitt 42 dem Patienten P Blut entnehmen und über den Bluteingang 2.3 dem Dialysator 2 zuführen kann. In dem arteriellen Schlauchabschnitt 42 sind in Strömungsrichtung eine arterielle Schlauchklemme 41, ein arterieller Hämatokrit-Sensor oder HCT-Sensor 44 zur Erfassung eines OxyhämoglobinAnteils $SpO_{2B}$ in dem Blut, eine Blutpumpe 46 und ein Bluteingangs-Druck-Sensor 48 angeordnet. Nachdem im extrakorporalen Blutkreislauf 3 das Blut des Patienten P durch die Blutseite des Dialysators 2 geleitet wurde, wird es an dessen Blutausgang 2.4 entnommen und über einen venösen Schlauchabschnitt 50 dem Shunt S zugeführt. In dem venösen Schlauchabschnitt 50 sind ein Blutausgangs-Druck-Sensor 52 und eine venöse Schlauchklemme 43 angeordnet. In dem Dialysator 2 wird das Blut im Gegenstromverfahren zur Dialysierflüssigkeit geführt und von harnpflichtigen Bestandteilen und überschüssigem Wasser befreit und hiernach gereinigt dem Patienten P zurückgegeben/ zurückgeführt.

Gemäß Figur 1 hat die extrakorporale Blutbehandlungsmaschine 1 eine Entgasungseinheit 5 zur Bereitstellung von zumindest teilentgastem Reinstwasser, deren Eingang 58 mit einem Versorgungsanschluss einer Umkehrosmose-Anlage (nicht dargestellt) verbunden ist und an dem gashaltiges Reinstwasser ansteht.

Ihr Eingang 58 ist über einen Reinstwasserzulauf 60 mit einem Entgasungstank 62 der Entgasungseinheit 5 verbunden, wobei in dem Reinstwasserzulauf 60 ein einstellbares Druckreduzierventil 64, insbesondere zum Einstellen eines Zulaufdrucks, und ein Absperrventil 66, insbesondere zum Sperren/ Aufsteuern des Reinstwasserzulaufs 60, angeordnet sind. Der Reinstwasserzulauf 60 mündet in eine Vorlaufkammer 68 des Entgasungstanks 62. Die Vorlaufkammer 68 hat zwei Schwimmerschalter 70, 72, einen unteren 70 und einen oberen 72, die mit einem Schwimmer 74 zusammenwirken und mit der Steuereinheit 54 signalverbunden sind.

Bodenseitig an der Vorlaufkammer 68 setzt ein Entgasungskanal 76 an, der die Vorlaufkammer 68 über eine offenbarungsgemäß mit kontinuierlich verstellbarem Drosselquerschnitt ausgestaltete Drosseleinrichtung 78 - die gemäß dem Ausführungsbeispiel als elektromagnetisch betätigbares Proportionalventil ausgestaltet ist - fluidisch mit einer Entgasungskammer 80 verbindet. Im Entgasungskanal 76 ist stromabwärts der Drosseleinrichtung 78 eine Druckerfassungseinheit 81 zur Erfassung des Entgasungs-Drucks $p_E$ angeordnet. In fluidisch paralleler Schaltung zur verstellbaren Drosseleinrichtung 78 ist eine feste Drossel 82 vorgesehen, über die ein paralleler Strömungspfad ausgebildet werden kann, wenn die verstellbare Drosseleinrichtung 78 zugesteuert ist.

Der Entgasungskanal 76 mündet bodenseitig in die Entgasungskammer 80. An einem Scheitel der Entgasungskammer 80 entspringt ein Abführabschnitt 84a eines Verbindungskanals 84, der über eine Förderpumpe 86 in eine Heizkammer 88 des Entgasungstanks 62 führt. Durch die Heizkammer 88 verläuft der Verbindungskanal 84 dann wendelförmig und tritt wiederum an einem Scheitel der Heizkammer 88 mit einem Rückführungsabschnitt 84b aus. Dieser mündet schließlich wieder in eine Beruhigungskammer 90 des Entgasungstanks 62 ein, welche zwischen der Vorlaufkammer 68 und der Heizkammer 88 angeordnet ist. Der Rückführungsabschnitt 84b des Verbindungskanals 84 weist einen Temperatursensor 92 und ein Sperrventil 94 auf.

Bodenseitig tritt aus der Beruhigungskammer 90 ein Bereitstellungskanal 96 aus, der über ein Sperrventil 98 mit dem bereits genannten Eingang 20 der Mischeinheit 6 verbunden ist.

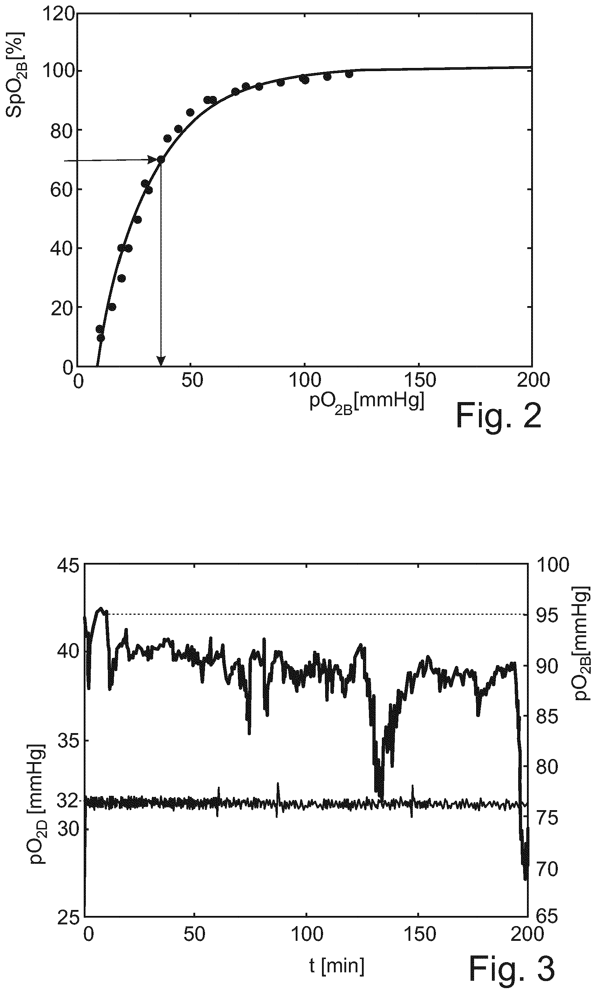

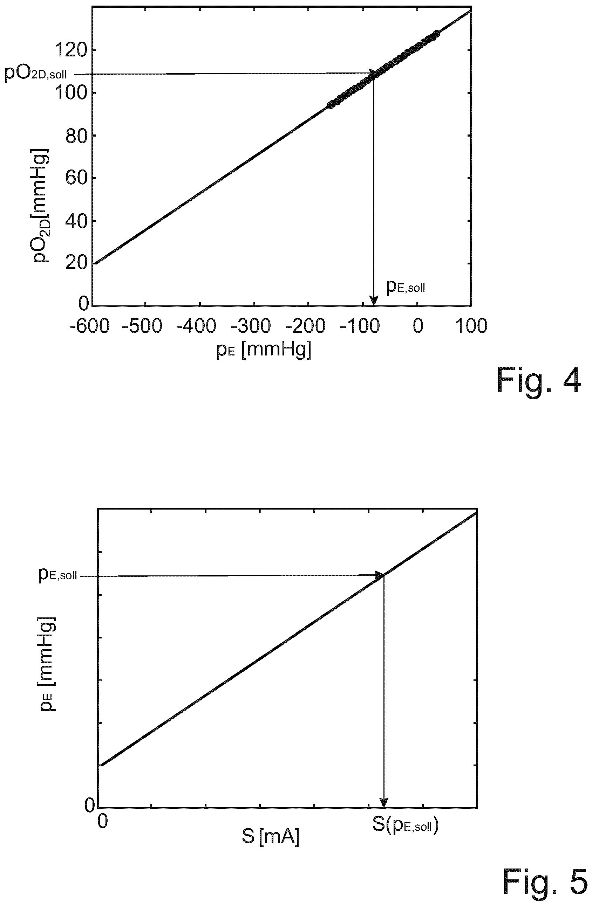

Anhand Figur 3 soll der offenbarungsgemäße Grundgedanke - nämlich den Sauerstoffgehalt in dem Blut durch Einstellen des Sauerstoffgehaltes in der frischen Dialysierflüssigkeit zu beeinflussen - veranschaulicht werden. Zur Erstellung des Graphen gemäß Figur 3 werden offenbarungsgemäß eine erste Korrelation, die vom Graphen der Figur 2 repräsentiert wird, und eine zweite Korrelation, die vom Graphen gemäß Figur 4 repräsentiert wird, herangezogen.

Figur 2 zeigt die erste Korrelation des Sauerstoffgehalts $pO_{2D}$ in dem Blut des extrakorporalen Blutkreislaufs 3 mit dem von einer Sensoreinheit 44 in dem Blut des extrakorporalen Blutkreislaufs 3 erfassten Blutbestandteil, das heißt mit dem Oxyhämoglobinanteil $SpO_{2B}$. Figur 4 zeigt die zweite Korrelation des Entgasungs-Drucks $p_E$ des Reinstwassers mit dem Sauerstoffgehalt $pO_{2D}$ in dem Reinstwasser oder der frischen Dialysierflüssigkeit. Beide Korrelationen sind in dem Speicher 56 der Steuereinheit 54 und von der Steuereinheit 54 aufrufbar abgelegt.

Gemäß der in Figur 2 dargestellten, ersten Korrelation ist auf der X-Achse der Sauerstoff-Partialdruck $pO_{2D}$ in dem Blut (in mmHg) und auf der Y-Achse der Oxyhämoglobinanteil $SpO_{2B}$ in dem Blut (in %) aufgetragen. Die erste Korrelation ist in dem Speicher 56 über die Steuereinheit 54 aufrufbar abgelegt. Mit Aufruf der ersten Korrelation ist die Steuereinheit 54 in der Lage, aus dem von der Sensoreinheit 44 prozentual ermittelten Oxyhämoglobinanteil $SpO_{2B}$ in dem Blut den Sauerstoff-Partialdruck $pO_{2D}$ in dem Blut zu ermitteln.

Die erste Korrelation basiert dabei auf empirisch ermittelte Daten. Die erste Korrelation des Oxyhämoglobinanteils $SpO_{2B}$ in dem Blut mit dem Sauerstoff-Partialdruck $pO_{2D}$ in dem Blut ist vorzugsweise in folgender Form im Speicher 56 aufrufbar abgelegt:

$$pO_{2B}(SpO_{2B}) = a * e^{(b*SpO_{2B})} + c * e^{(d*SpO_{2B})} \qquad (1)$$

Gemäß der in Figur 4 dargestellten, zweiten Korrelation ist auf der X-Achse der Entgasungs-Druck $p_E$ und auf der Y-Achse der Sauerstoff-Partialdruck $pO_{2D}$ in dem zumindest teilentgasten Reinstwasser oder der frischen Dialysierflüssigkeit aufgetragen (beide in mmHg). Auch die zweite Korrelation gemäß Figur 4 ist in dem Speicher 56 über die Steuereinheit 54 aufrufbar abgelegt. Mit Aufruf der zweiten Korrelation ist die Steuereinheit 54 in der Lage, aus dem von der Druckerfassungseinheit 81 erfassten Entgasungsdruck $p_E$ den zugehörigen Sauerstoff-Partialdruck $pO_{2D}$ in dem zumindest teilentgasten Reinstwasser oder der frischen Dialysierflüssigkeit zu ermitteln, sowie umgekehrt, aus einem von ihr ermittelten Soll-Sauerstoff-Partialdruck $pO_{2D,soll}$ in dem zumindest teilentgasten Reinstwasser oder der frischen Dialysierflüssigkeit einen Soll-Entgasungs-Druck $p_{E,soll}$ des zumindest teilentgasten Reinstwassers zu ermitteln.

Die zweite Korrelation des Sauerstoff-Partialdrucks $pO_{2D}$ in dem zumindest teilentgasten Reinstwasser oder der frischen Dialysierflüssigkeit mit dem Entgasungs-Druck $p_E$ ist vorzugsweise in folgender Form im Speicher 56 aufrufbar abgelegt:

$$pO_{2D}(p_E) = a * p_E + b \qquad (2)$$

Figur 3 führt die mit Hilfe der beiden Korrelationen über die Steuereinheit 54 ermittelten, zeitlichen Verläufe der Sauerstoff-Partialdrücke in dem Blut $pO_{2D}$ und in der frischen Dialysierflüssigkeit $pO_{2D}$ für eine exemplarische Blutbehandlung zusammen. Zu Beginn (bei t = 0) und über die gesamte dargestellte Behandlungsdauer (bis t = 200) liegt der Sauerstoff-Partialdruck $pO_{2D}$ in der frischen Dialysierflüssigkeit bei etwa 32mmHg. In dem Blut hingegen liegt der Sauerstoff-Partialdruck $pO_{2D}$ bei etwa 91 mmHg. Damit liegt gemäß Figur 3 ein deutlicher Gradient des Sauerstoffgehaltes von dem Blut über die Membran 2.5 des Dialysators 2 in Richtung der Dialysierflüssigkeit vor. Die Porengröße einer Dialysemembran liegt etwa zwischen 1.8nm (LowFlux) bis 3.3nm (HighFlux). Der Atomradius von Sauerstoff liegt hingegen bei etwa 0.06nm. Grundsätzlich ist somit der in der Dialysierflüssigkeit gelöste Sauerstoff über die Membran 2.5 des Dialysators 2 gemäß Figur 1 gut permeabel, so dass entsprechend der herrschenden Differenz der Sauerstoff-Partialdrücke $pO_{2B}$, $pO_{2D}$ mit einem Sauerstoff-Transport zu rechnen ist. Im vorliegenden, exemplarischen Fall gemäß Figur 3 ist tatsächlich zu beobachten, dass der Sauerstoff-Partialdruck $pO_{2D}$ in dem Blut über die Behandlungsdauer t abnimmt, von etwa 91 mmHg auf etwa 86 mmHg.

Da, wie bereits erwähnt, der Sauerstoff-Partialdruck $pO_{2D}$ in der frischen Dialysierflüssigkeit bereits zu Anfang und über die gesamte Dauer t erheblich unter dem Sauerstoff-Partialdruck $pO_{2D}$ in dem Blut liegt, ist festzustellen, dass das in Figur 3 dargestellte Abfallen des Sauerstoff-Partialdrucks $pO_{2D}$ in dem Blut auf einen Übertritt von Sauerstoff von dem Blut in die Dialysierflüssigkeit zurückgeht.

Im Umkehrschluss ist es gemäß der Offenbarung möglich, diesen Übertritt durch den Wert des Sauerstoff-Partialdrucks $pO_{2D}$ in der frischen Dialysierflüssigkeit zu beeinflussen, worin der Grundgedanke der vorliegenden Offenbarung liegt, nämlich insbesondere den Übertritt zu senken oder ggf. zu verhindern oder ggf. sogar umzukehren.

Es ist somit festzustellen, dass extrakorporalen Blutbehandlungsmaschinen durch die offenbarungsgemäße Beeinflussung des Sauerstoff-Partialdrucks $pO_{2D}$ in der frischen Dialysierflüssigkeit eine neue Funktionalität - nämlich den Sauerstoffgehalt in dem Blut durch Einstellen des Sauerstoffgehaltes in der frischen Dialysierflüssigkeit zu beeinflussen - hinzugefügt werden kann.

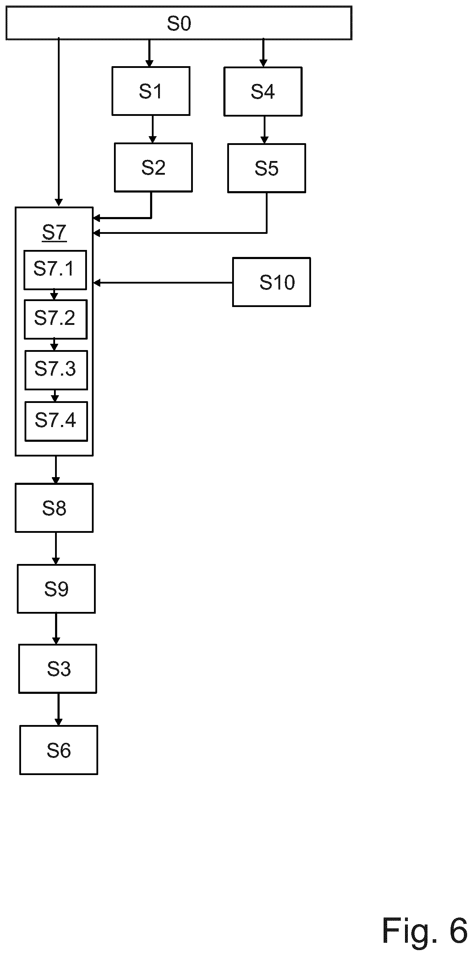

Es folgt die Beschreibung der offenbarungsgemäßen Entgasung des gashaltigen Reinstwassers über die Entgasungseinheit 62 anhand der Figuren 1 bis 5 und insbesondere anhand der Figur 6, welche das offenbarungsgemäße, in der Steuereinheit 54 computerimplementierte Steuerungsverfahren zeigt.

Gemäß Figur 1 steht an dem Eingang 58 der Entgasungseinheit 62 von der Umkehrosmose-Anlage (nicht dargestellt) bereitgestelltes, gashaltiges Reinstwasser an. Dieses enthält unter Normalbedingungen beträchtliche Mengen an gelösten Gasen. Bei einer Zulauftemperatur von beispielsweise 20°C enthält es unter Normaldruck bis zu $19cm^3/l$ gelöstes Gas. Die Löslichkeit von Gas in dem Reinstwasser wird grundsätzlich durch das Henry-Gesetz beschrieben. Demnach nimmt die Kapazität des Reinstwassers, Gas zu lösen mit dem Druck zu und mit der Temperatur ab. Wird ein mehrkomponentiges Gas, wie beispielsweise Luft, in einer Flüssigkeit gelöst, so sind für die in der Flüssigkeit gelösten Gasmengen die einzelnen Partialdrücke der Komponenten von Bedeutung. Hierzu sei auf die weiter oben bereits beschriebene, zweite Korrelation verwiesen. Etwa ein Drittel des in der Flüssigkeit gelösten Gases entfällt dabei auf den Sauerstoff, welcher messtechnisch leicht zugänglich ist.

Gemäß Figur 1 steuert die Steuereinheit 54 das Absperrventil 66 auf, solange der obere Schwimmschalter 72 nicht schaltet. Entsprechend strömt gashaltiges Reinstwasser in die Vorlaufkammer 68 und weiter über den Entgasungskanal 76 der Drosselrichtung 78 zu. Um die Beschreibung zu vereinfachen sei angenommen, dass in dem Entgasungskanal 76 ein kontinuierlicher Reinstwasser-Fluss vorliegt.

Das computerimplementierte Steuerverfahren wird mit einem Schritt **S0** gestartet oder initialisiert. Der Start oder die Initialisierung kann beispielsweise automatisiert mit Inbetriebnahme der Blutbehandlungsmaschine 1 oder durch eine manuelle Eingabe eines Bedienpersonals über eine Bedienschnittstelle der Blutbehandlungsmaschine 1 erfolgen.

Nach Inbetriebnahme erfolgen fortwährend die Schritte Ermitteln **S1** des Oxyhämoglobinanteils $SpO_{2B}$ in dem im extrakorporalen Blutkreislauf 3 geführten, arteriellen Blut, über die Sensoreinheit 44, und Bereitstellen **S2** des ermittelten Oxyhämoglobinanteils $SpO_{2B}$ an die Steuereinheit 54, sowie die Schritte Erfassen **S4** des Entgasungs-Drucks $p_\varepsilon$, über die Druckerfassungseinheit 81 im Entgasungskanal 76 stromabwärts der Drosseleinrichtung 78 und Bereitstellen **S5** des erfassten Entgasungs-Drucks $p_E$ an die Steuereinheit 54.

Mittels der Drosseleinrichtung 78 erfolgt permanent ein Schritt Drosseln **S3** des gashaltigen Reinstwassers auf den Entgasungs-Druck $p_\varepsilon$, sodass das Reinstwasser dem Entgasungs-Druck $p_E$ entsprechend voll entgast oder teilentgast wird.

Im Anschluss strömt das entgaste oder teilentgaste Reinstwasser durch die Entgasungskammer 80, an deren Oberflächenstruktur sich das entlöste Gas vom Reinstwasser effizient trennt. Aus der Entgasungskammer 80 tritt das entgaste oder teilentgaste Reinstwasser aus und strömt über den Verbindungskanal 84 und die Heizkammer 88, wo es erwärmt werden kann, zurück in die Beruhigungskammer 90. Hier wird es bodenseitig abgezogen und strömt über das Sperrventil dem Ausgang der Entgasungseinheit 5 und damit der Mischeinheit 6 zu.

In der Mischeinheit 6 erfolgt dann der Schritt Mischen **S6** des entgasten oder teilentgasten Reinstwassers mit wenigstens einem Konzentrat zu der frischen Dialysierflüssigkeit. Die frische Dialysierflüssigkeit tritt in Folge über den Dialysierflüssigkeitszulauf 4 und den Dialysierflüssigkeitseingang 2.1 in den Dialysator 2 ein.

Mit dem Steuerungsverfahren gemäß Figur 6 erfolgen über die Steuereinheit 54 offenbarungsgemäß die Schritte Ermitteln **S7** eines Ansteuersignals S zum Ansteuern der Drosselvorrichtung 78 und Ansteuern **S8** der Drosselvorrichtung 78 mit dem Ansteuersignal S, woraus der Schritt Verstellen **S9** des Drosselquerschnitts gemäß dem Ansteuersignal S resultiert.

Der Schritt Ermitteln **S7** des Ansteuersignals S zum Ansteuern der Drosselvorrichtung 78 erfolgt dabei offenbarungsgemäß in Teilschritten **S7.1** bis **S7.4**. Grundlage für die Ausführung dieser Teilschritte ist, dass in dem Speicher 56 die beiden vorbeschriebenen Korrelationen gemäß Figur 2 und 4, eine dritte Korrelation des Entgasungsdrucks $p_E$ mit dem Ansteuersignal S gemäß Figur 5, sowie eine vorbestimmte Soll-Sauerstoff-Partialdruck-Differenz $\Delta pO_{2,soll}$ zwischen dem Sauerstoff-Partialdruck $pO_{2D}$ in der frischen Dialysierflüssigkeit und dem Sauerstoff-Partialdruck $pO_{2D}$ in dem Blut abgelegt sind.

Zunächst erfolgt ein Schritt Aufrufen **S7.1** der ersten Korrelation und Ermitteln des Sauerstoff-Partialdrucks $pO_{2D}$ in dem Blut in Abhängigkeit des von der Sensoreinheit 44 ermittelten Oxyhämoglobinanteils $SpO_{2B}$ in dem Blut, über die Steuereinheit 54 (vgl. Figur 2). Darauf aufbauend erfolgt ein Schritt Ermitteln **S7.2** eines Soll-Sauerstoff-Partialdrucks $pO_{2D,soll}$ in der frischen Dialysierflüssigkeit als Summe aus dem ermittelten Sauerstoff-Partialdruck $pO_{2D}$ in dem Blut und der vorbestimmten Soll-Sauerstoff-Partialdruck-Differenz $\Delta pO_{2,soll}$, über die Steuereinheit 54. Es folgt ein Schritt Aufrufen **S7.3** der zweiten Korrelation und Ermitteln eines Soll-Entgasungsdrucks $p_{E,soll}$ in Abhängigkeit des ermittelten Soll-Sauerstoff-Partialdrucks $pO_{2D,soll}$ in der frischen Dialysierflüssigkeit, über die Steuereinheit 54 (vgl. Figur 4). Im Anschluss erfolgt ein Schritt Aufrufen **S7.4** der dritten Korrelation und Ermitteln des Ansteuersignals S in Abhängigkeit des ermittelten Soll-Entgasungsdrucks $p_{E,soll}$, über die Steuereinheit 54 (vgl. Figur 5).

Damit ist für den Schritt **S7** das Ansteuersignal S derart ermittelt, dass das gashaltige Reinstwasser auf einen Entgasungs-Druck $p_E$ gedrosselt wird, der zu einer Teilentgasung führt, nach welcher die aus dem teilentgasten Reinstwasser gemischte, frische Dialysierflüssigkeit den Soll-Sauerstoff-Partialdrucks $pO_{2D,soll}$ aufweist.

Ergänzend weist das Steuerungsverfahren einen permanent, punktuell, und/oder periodisch durchgeführten Schritt Ermitteln **S10** einer Abweichung der Ist-Sauerstoff-Partialdruck-Differenz $\Delta pO_{2,Ist}$ von der vorbestimmten Sauer-stoff-Partialdruck-Differenz $\Delta pO_{2,soll}$ und Anpassen des Ansteuersignals S an die Abweichung auf.

**EP 4 591 897 A1**

Bezugszeichenliste

| | |
|---|---|
| 1 | Extrakorporale Blutbehandlungsmaschine |
| 2 | Dialysator |
| 2.1 | Dialysierflüssigkeitseingang |
| 2.2 | Dialysatausgang |
| 2.3 | Bluteingang |
| 2.4 | Blutausgang |
| 2.5 | semipermeable Membran |
| 3 | extrakorporaler Blutkreislauf |
| 4 | Dialysierflüssigkeitszulauf |
| 5 | Entgasungseinheit |
| 6 | Mischeinheit |
| 8 | erste Quelle saures Konzentrat |
| 10 | zweite Quelle basisches Konzentrat |
| 12 | erste Fördervorrichtung |
| 14 | zweite Fördervorrichtung |
| 16 | erste Messvorrichtung |
| 18 | zweite Messvorrichtung |
| 20 | Reinstwassereingang |
| 22 | dritte Fördervorrichtung |
| 24 | Bilanziervorrichtung |
| 26 | erstes Ventil |
| 28 | Dialysatablauf |
| 30 | Entsorgungsausgang |
| 32 | Erfassungseinheit |
| 34 | zweites Ventil |
| 38 | Bypass-Strömungspfad |
| 40 | drittes Ventil |
| 41 | arterielle Schlauchklemme |
| 42 | arterieller Schlauchabschnitt |
| 43 | venöse Schlauchklemme |
| 44 | Blutbestandteil-Sensor |
| 46 | Blutpumpe |
| 48 | Bluteingangs-Druck-Sensor |
| 50 | venöser Schlauchabschnitt |
| 52 | Blutausgangs-Druck-Sensor |
| 54 | Steuereinheit |
| 56 | Speicher |
| 58 | Eingang Entgasungseinheit |
| 60 | Reinstwasserzulauf |
| 62 | Entgasungstank |
| 64 | Druckreduzierventil |
| 66 | Absperrventil |
| 68 | Vorlaufkammer |
| 70, 72 | Schwimmerschalter |
| 74 | Schwimmer |
| 76 | Entgasungskanal |
| 78 | verstellbare Drosseleinrichtung |
| 80 | Entgasungskammer |
| 81 | Druckerfassungseinheit |
| 82 | feste Drossel |
| 84 | Verbindungskanal |
| 86 | Förderpumpe |
| 88 | Heizkammer |
| 90 | Beruhigungskammer |

| 92 | Temperatursensor |
|---|---|
| 94 | Sperrventil |
| 96 | Bereitstellungskanal |
| 98 | Sperrventil |

| S0 | Start Steuerverfahren |
|---|---|
| S1 | Schritt Ermitteln Blutbestandteil |
| S2 | Schritt Bereitstellen Blutbestandteil |
| S3 | Schritt Drosseln gashaltiges Reinstwasser |
| S4 | Schritt Erfassen Entgasungs-Druck |
| S5 | Schritt Bereitstellen Entgasungs-Druck |
| S6 | Schritt Mischen frische Dialysierflüssigkeit |
| S7 | Schritt Ermitteln Ansteuersignal |
| S7.1 | Schritt Ermitteln Sauerstoffgehalt Blut |
| S7.2 | Schritt Ermitteln Soll-Sauerstoffgehalt Dialysierflüssigkeit |
| S7.3 | Schritt Ermitteln Soll-Entgasungsdruck |
| S7.4 | Schritt Ermitteln Ansteuersignal aus Soll-Entgasungsdruck |
| S8 | Schritt Ansteuern Drosseleinrichtung |
| S9 | Schritt Verstellen Drosselquerschnitt |
| S10 | Schritt Anpassen Soll-Partialdruck-Differenz Sauerstoffgehalt |

| P | Patient |
|---|---|
| S | Shunt |
| $SpO_{2B}$ | Blutbestandteil |
| $pO_{2D}$ | Sauerstoffgehalt Blut |
| $pO_{2D}$ | Sauerstoffgehalt Reinstwasser/ frische Dialysierflüssigkeit |
| $pO_{2D,soll}$ | Soll-Sauerstoffgehalt Reinstwasser/ frische Dialysierflüssigkeit |
| $\Delta pO_{2,soll}$ | vorbestimmte Differenz |
| $p_E$ | Entgasungs-Druck |
| $p_{E,soll}$ | Soll-Entgasungsdruck |
| S | Ansteuersignal |

**Patentansprüche**

1. Extrakorporale Blutbehandlungsmaschine (1), insbesondere Dialysemaschine, für eine extrakorporale Blutbehandlung von Blut eines Patienten (P), aufweisend:

   - einen Dialysator (2),
   - einen extrakorporalen Blutkreislauf (3), welcher über einen Bluteingang (2.3) und einen Blutausgang (2.4) des Dialysators (2) durch den Dialysator (2) verläuft, mit einer Sensoreinheit (44), die dafür angepasst ist, einen Blutbestandteil ($SpO_{2B}$) in dem Blut zu ermitteln, insbesondere einen Oxyhämoglobinanteil des Blutes, der mit einem Sauerstoffgehalt ($pO_{2B}$) in dem Blut, insbesondere einem Sauerstoff-Partialdruck in dem Blut, korreliert, wobei die Sensoreinheit (44) vorzugsweise stromaufwärts des Bluteingangs (2.3) angeordnet ist,
   - eine Entgasungseinheit (5) mit einer Drosseleinrichtung (78), die dafür angepasst ist, gashaltiges Reinstwasser auf einen Entgasungs-Druck ($p_E$) zu drosseln, um an einem Ausgang (96) der Entgasungseinheit (5) zumindest teilentgastes Reinstwasser bereitzustellen, mit einer Druckerfassungseinheit (81), die dafür angepasst ist, den Entgasungs-Druck ($p_E$) zu erfassen, wobei die Druckerfassungseinheit (81) insbesondere stromabwärts der Drosseleinrichtung (78) angeordnet ist,
   - eine Mischeinheit (6), die dafür angepasst ist, das zumindest teilentgaste Reinstwasser mit wenigstens einem Konzentrat zu einer frischen Dialysierflüssigkeit zu mischen, und
   - eine Steuereinheit (54), die mit der Sensoreinheit (44), der Druckerfassungseinheit (81) und der Drosseleinrichtung (78) signalverbunden ist, **dadurch gekennzeichnet, dass**

   die Drosseleinrichtung (78) mit einem kontinuierlich verstellbaren Drosselquerschnitt ausgestaltet ist, welcher in Abhängigkeit eines Ansteuersignals (S) der Steuereinheit (54), vorzugsweise proportional zu dem Ansteuersignal (S), kontinuierlich verstellbar ist.

2. Extrakorporale Blutbehandlungsmaschine (1) nach Anspruch 1, **dadurch gekennzeichnet, dass** die Steuereinheit (54) dafür angepasst ist, das Ansteuersignal (S) zu ermitteln, um den Sauerstoffgehalt ($pO_{2B}$) in dem Blut zu heben, konstant zu halten und/oder zu senken.

3. Extrakorporale Blutbehandlungsmaschine (1) nach Anspruch 1 oder 2, **dadurch gekennzeichnet, dass** die Steuereinheit (54) dafür angepasst ist, das Ansteuersignal (S) zu ermitteln, um einen Sauerstoffgehalt ($pO_{2D}$) in dem zumindest teilentgasten Reinstwasser oder der frischen Dialysierflüssigkeit, insbesondere einen Sauerstoff-Partialdruck in dem zumindest teilentgasten Reinstwasser oder der frischen Dialysierflüssigkeit, in Abhängigkeit des Sauerstoffgehaltes ($pO_{2B}$) in dem Blut einzustellen, insbesondere zu heben, konstant zu halten und/oder zu senken.

4. Extrakorporale Blutbehandlungsmaschine (1) nach einem der vorhergehenden Ansprüche, **dadurch gekennzeichnet, dass** die Steuereinheit (54) dafür angepasst ist, das Ansteuersignal (S) zu ermitteln, um eine vorbestimmte Differenz ($\Delta pO_{2,soll}$) zwischen einem Sauerstoffgehalt ($pO_{2D}$) in dem zumindest teilentgasten Reinstwasser oder der frischen Dialysierflüssigkeit, insbesondere einem Sauerstoff-Partialdruck in dem zumindest teilentgasten Reinstwasser oder der frischen Dialysierflüssigkeit, und dem Sauerstoffgehalt ($pO_{2B}$) in dem Blut einzustellen, insbesondere konstant zu halten.

5. Extrakorporale Blutbehandlungsmaschine (1) nach einem der vorhergehenden Ansprüche, **dadurch gekennzeichnet, dass** in einem Speicher (56) der Blutbehandlungsmaschine (1), vorzugsweise in einem Speicher (56) der Steuereinheit (54), eine erste Korrelation des Sauerstoffgehalts ($pO_{2B}$) in dem Blut mit dem Blutbestandteil ($SpO_{2B}$) abgelegt ist, und dass die Steuereinheit (54) dafür angepasst ist, die erste Korrelation aufzurufen und den Sauerstoffgehalt ($pO_{2B}$) in dem Blut in Abhängigkeit des von der Sensoreinheit (44) ermittelten Blutbestandteils ($SpO_{2B}$) zu ermitteln.

6. Extrakorporale Blutbehandlungsmaschine (1) nach Anspruch 4 und 5, **dadurch gekennzeichnet, dass** die Steuereinheit (54) dafür angepasst ist, einen Soll-Sauerstoffgehalt ($pO_{2D,soll}$) in dem zumindest teilentgasten Reinstwasser oder der frischen Dialysierflüssigkeit aus dem ermittelten Sauerstoffgehalt ($pO_{2B}$) in dem Blut und der vorbestimmten Differenz ($\Delta pO_{2,soll}$), insbesondere als deren Summe, zu ermitteln.

7. Extrakorporale Blutbehandlungsmaschine (1) nach Anspruch 6, **dadurch gekennzeichnet, dass** in dem Speicher (56) eine zweite Korrelation des Sauerstoffgehaltes ($pO_{2D}$) in dem zumindest teilentgasten Reinstwasser oder der frischen Dialysierflüssigkeit mit dem Entgasungsdruck ($p_E$) abgelegt ist, und dass die Steuereinheit (54) dafür angepasst ist, die zweite Korrelation aufzurufen und einen Soll-Entgasungsdruck ($p_{E,soll}$) in Abhängigkeit des ermittelten Soll-Sauerstoffgehaltes ($pO_{2D.soll}$) in dem zumindest teilentgasten Reinstwasser oder der frischen Dialysierflüssigkeit zu ermitteln.

8. Extrakorporale Blutbehandlungsmaschine (1) nach Anspruch 7, **dadurch gekennzeichnet, dass** in dem Speicher (56) eine dritte Korrelation des Entgasungsdrucks ($p_E$) mit dem Ansteuersignal (S) abgelegt ist, und dass die Steuereinheit (54) dafür angepasst ist, die dritte Korrelation aufzurufen und das Ansteuersignal (S) in Abhängigkeit des ermittelten Soll-Entgasungsdrucks ($p_{E,soll}$) zu ermitteln und die Drosseleinrichtung (78) mit dem Ansteuersignal (S) anzusteuern.

9. Extrakorporale Blutbehandlungsmaschine (1) zumindest nach Anspruch 4, **dadurch gekennzeichnet, dass** die Steuereinheit (54) dafür angepasst ist, eine Abweichung von der vorbestimmten Differenz ($\Delta pO_{2,soll}$) permanent, punktuell, und/oder periodisch zu ermitteln und das Ansteuersignal (S) in Abhängigkeit der Abweichung anzupassen.

10. Computerimplementiertes Steuerverfahren für eine extrakorporale Blutbehandlungsmaschine (1) mit einem Dialysator (2), insbesondere einer Blutbehandlungsmaschine (1) nach einem der vorstehenden Ansprüche, mit Schritten:

    - Ermitteln (S1) eines Blutbestandteils ($SpO_{2B}$) in dem Blut, insbesondere eines Oxyhämoglobinanteils, welcher mit einem Sauerstoffgehalt ($pO_{2B}$) in dem Blut, insbesondere einem Sauerstoff-Partialdruck in dem Blut, korreliert, über eine Sensoreinheit (44) in einem extrakorporalen Blutkreislauf (3) der Blutbehandlungsmaschine (1),
    - Bereitstellen (S2) des ermittelten Blutbestandteils ($SpO_{2B}$) an eine Steuereinheit (54);
    - Drosseln (S3) von gashaltigem Reinstwasser auf einen Entgasungs-Druck ($p_E$), um zumindest teilentgastes Reinstwasser bereitzustellen, über eine Drosseleinrichtung (78),
    - Erfassen (S4) des Entgasungs-Drucks ($p_E$), über eine Druckerfassungseinheit (81),
    - Bereitstellen (S5) des Entgasungs-Drucks ($p_E$) an die Steuereinheit (54);
    - Mischen (S6) des zumindest teilentgasten Reinstwassers mit wenigstens einem Konzentrat zu einer frischen Dialysierflüssigkeit, über eine Mischeinheit (6), **gekennzeichnet durch** Schritte
- Ermitteln (S7) eines Ansteuersignals (S) zum Ansteuern der Drosselvorrichtung (78), die mit einem in Abhängigkeit des Ansteuersignals (S) verstellbaren, vorzugsweise proportional zu dem Ansteuersignal (S) verstellbaren, Drosselquerschnitt ausgestaltet ist, über die Steuereinheit (54);
- Ansteuern (S8) der Drosselvorrichtung (78) mit dem Ansteuersignal (S), über die Steuereinheit (54); und
- Verstellen (S9) des Drosselquerschnitts gemäß dem Ansteuersignal (S).

11. Computerimplementiertes Steuerverfahren nach Anspruch 10, **dadurch gekennzeichnet, dass** das Ansteuersignal (S) über die Steuereinheit (54) derart ermittelt wird, dass der Sauerstoffgehalt ($pO_{2B}$) in dem Blut wahlweise gehoben, konstant gehalten und/oder gesenkt wird.

12. Computerimplementiertes Steuerverfahren nach Anspruch 10 oder 11, **dadurch gekennzeichnet, dass** das Ansteuersignal (S) über die Steuereinheit (54) derart ermittelt wird, dass ein Sauerstoffgehalt ($pO_{2D}$) in dem zumindest teilentgasten Reinstwasser oder der frischen Dialysierflüssigkeit, insbesondere ein Sauerstoff-Partialdruck in dem zumindest teilentgasten Reinstwasser oder der frischen Dialysierflüssigkeit, in Abhängigkeit des Sauerstoffgehaltes ($pO_{2B}$) in dem Blut wahlweise gehoben, konstant gehalten und/oder gesenkt wird.

13. Computerimplementiertes Steuerverfahren nach einem der Ansprüche 10 bis 12, **dadurch gekennzeichnet, dass** das Ansteuersignal (S) über die Steuereinheit (54) derart ermittelt wird, dass eine vorbestimmte Differenz ($\Delta pO_{2,soll}$) zwischen einem Sauerstoffgehalt ($pO_{2D}$) in dem zumindest teilentgasten Reinstwasser oder der frischen Dialysierflüssigkeit, insbesondere einem Sauerstoff-Partialdruck in dem zumindest teilentgasten Reinstwasser oder der frischen Dialysierflüssigkeit, und dem Sauerstoffgehalt ($pO_{2B}$) in dem Blut eingestellt, insbesondere konstant gehalten, wird.

14. Computerimplementiertes Steuerverfahren nach Anspruch 13, wobei in einem Speicher (56) der Blutbehandlungsmaschine (1), vorzugsweise in einem Speicher (56) der Steuereinheit (54), eine erste Korrelation des Sauerstoffgehalts ($pO_{2B}$) in dem Blut mit dem Blutbestandteil ($SpO_{2B}$), eine zweite Korrelation des Sauerstoffgehalts ($pO_{2D}$) in dem zumindest teilentgasten Reinstwasser oder der frischen Dialysierflüssigkeit mit dem Entgasungsdruck ($p_E$), eine dritte Korrelation des Entgasungsdrucks ($p_E$) mit dem Ansteuersignal (S), sowie die vorbestimmte Differenz ($\Delta pO_{2,soll}$) abgelegt sind, **gekennzeichnet durch** Schritte zum Ermitteln (S7) des Ansteuersignals (S):

   - Aufrufen (S7.1) der ersten Korrelation und Ermitteln des Sauerstoffgehalts ($pO_{2B}$) in dem Blut in Abhängigkeit des von der Sensoreinheit (44) erfassten Blutbestandteils ($SpO_{2B}$), über die Steuereinheit (54);
   - Ermitteln (S7.2) eines Soll-Sauerstoffgehalts ($pO_{2D,soll}$) in dem zumindest teilentgasten Reinstwasser oder der frischen Dialysierflüssigkeit aus dem ermittelten Sauerstoffgehalt ($pO_{2B}$) in dem Blut und der vorbestimmten Differenz ($\Delta pO_{2,soll}$), insbesondere als deren Summe, über die Steuereinheit (54);
   - Aufrufen (S7.3) der zweiten Korrelation und Ermitteln eines Soll-Entgasungsdrucks ($p_{E,soll}$) in Abhängigkeit des ermittelten Soll-Sauerstoffgehalts ($pO_{2D,soll}$) in dem zumindest teilentgasten Reinstwasser oder der frischen Dialysierflüssigkeit, über die Steuereinheit (54); und
   - Aufrufen (S7.4) der dritten Korrelation und Ermitteln des Ansteuersignals (S) in Abhängigkeit des ermittelten Soll-Entgasungsdrucks ($p_{E,soll}$), über die Steuereinheit (54).

15. Computerprogramm, umfassend Befehle, die bei einer Ausführung durch einen Computer diesen veranlassen die Verfahrensschritte des Steuerverfahrens nach einem der Ansprüche 10 bis 14 auszuführen.

Fig. 1

EP 4 591 897 A1

Fig. 2

Fig. 3

Fig. 4

Fig. 5

Fig. 6

Europäisches
Patentamt

European
Patent Office

Office européen
des brevets

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2002/068015 A1 (POLASCHEGG HANS-DIETRICH [AT] ET AL) 6. Juni 2002 (2002-06-06) * Ansprüche 1,2 * * Absatz [0012] * * Absatz [0019] * - - - - - | 1-15 | INV. A61M1/16 |
| A | EP 2 717 938 B1 (FRESENIUS MEDICAL CARE DE GMBH [DE]) 19. August 2015 (2015-08-19) * Absatz [0007] * * Absatz [0016] * * Absatz [0065] * * Absatz [0072] - Absatz [0073]; Abbildung 1 * * Absatz [0106] * - - - - - | 1-15 | |

**RECHERCHIERTE SACHGEBIETE (IPC)**

A61M

Der vorliegende Recherchenbericht wurde für alle Patentansprüche erstellt

| Recherchenort | Abschlußdatum der Recherche | Prüfer |
|---|---|---|
| Den Haag | 22. Juni 2025 | Mata Vicente, Teresa |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes
  Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**
EP 25 15 3435

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-06-2025

| Im Recherchenbericht angeführtes Patentdokument | Datum der Veröffentlichung | Mitglied(er) der Patentfamilie | Datum der Veröffentlichung |
|---|---|---|---|
| US 2002068015 A1 | 06-06-2002 | DE 10047421 C1 | 02-01-2003 |
| | | EP 1192962 A1 | 03-04-2002 |
| | | US 2002068015 A1 | 06-06-2002 |
| EP 2717938 B1 | 19-08-2015 | CN 103608051 A | 26-02-2014 |
| | | CO 6811821 A2 | 16-12-2013 |
| | | DE 102011106111 A1 | 13-12-2012 |
| | | EP 2717938 A1 | 16-04-2014 |
| | | JP 6101995 B2 | 29-03-2017 |
| | | JP 2014523759 A | 18-09-2014 |
| | | US 2012316799 A1 | 13-12-2012 |
| | | WO 2012167929 A1 | 13-12-2012 |

EPO FORM P0461

Für nähere Einzelheiten zu diesem Anhang : siehe Amtsblatt des Europäischen Patentamts, Nr.12/82